# EUROPÄISCHE PATENTSCHRIFT

(11) **EP 1 484 086 B1**
(45) Veröffentlichungstag und Bekanntmachung des Hinweises auf die Patenterteilung: **10.11.2010**
(21) Anmeldenummer: 04011013.2
(22) Anmeldetag: 07.05.2004
(51) Int. Cl.: A61K 8/27, A61K 8/19, A61K 8/365, A61Q 19/00

(54) **Zubereitung zur Vorbeugung und Behandlung von Akne enthaltend Zinkgluconat und Kupfergluconat**
Composition for preventing and curing the acne comprising zinc gluconate and copper gluconate
Composition pour la prévention et le traitement de l'acné comprenant du gluconate de zinc et du gluconate de cuivre

(30) Priorität: 09.05.2003 DE 10320852
(43) Veröffentlichungstag der Anmeldung: 08.12.2004
(73) Patentinhaber: Dr. Scheller Cosmetics AG, 73054 Eislingen (DE)
(72) Erfinder: Scheller, Hans-Ulrich, 73054 Eislingen (DE)
(74) Vertreter: Gesthuysen, von Rohr & Eggert

(56) Entgegenhaltungen:
- EP-A- 1 444 973
- WO-A-03/013561
- WO-A-2004/022024
- FR-A- 2 765 105
- US-A- 4 428 933
- US-A1- 2002 009 506
- DATABASE WPI Section Ch, Week 200377 Derwent Publications Ltd., London, GB; Class B04, AN 2003-826265 XP002298897 & KR 2003 047 457 A (LG HOUSEHOLD & HEALTH CARE LTD) 18. Juni 2003 (2003-06-18)

## Beschreibung

Gegenstand der vorliegenden Erfindung sind bakteriostatische Zubereitungen zur Vorbeugung und Behandlung der Akne und unreiner Haut.

Mittel zur Behandlung der Akne sind beispielsweise bekannt aus der EP 0699432, in der Mittel enthaltend Thermal- und Mineralwasser beschrieben sind.

Hautunreinheiten, Pickel, Vorstufen der Akne und Akne sind die häufigsten Hautprobleme während der Pubertät und der Adoleszenz. Rund 80 % der Jugendlichen leiden mehr oder weniger stark an diesen Hautproblemen. Dabei wird vor allem das Gesicht betroffen, was für den jungen Menschen sowohl ein kosmetisches als auch ein psychisches Problem sein kann. Häufig verlaufen diese Probleme chronisch mit der Folge bleibender unschöner Narben.

Die drei häufigsten Ursachen, die zu Hautproblemen führen sind:
- erhöhte Talgproduktion, die durch Hormone ausgelöst wird,
- Verbindung des Talgabflusses durch Verstopfung (übermäßige Verhornung) des Follikelausganges,
- Entzündungen, verursacht durch bakterielle Infektionen, mit der Folge eventueller Narbenbildung.

Da insbesondere die Vorstadien der Akne kein klassisches Krankheitsbild, sondern ein kosmetisches Problem darstellen, kommt einer stadiengerechten kosmetischen topischen Behandlung besondere Bedeutung zu, die darauf abzielt, einen direkten Effekt auf die entzündungsauslösenden Bakterien, insbesondere das Propionibacterium, auszuüben.

Hierzu kommt entsprechend des Pathomechanismus der unreinen Haut die Therapie auf Komedolyse, Sebosuppressive und o. g. Bakteriostase.

### Komedolyse

Die Dermatologie setzt hier vorwiegend Retinoide ein, die nicht nur die Zwischenzellsubstanzen auflösen, sondern auch eine Abstoßung des Komedo bewirken. Außerdem ist der Einsatz von Salizylsäure bis zu einer Konzentration bis zu 5 % populär.

Die Kosmetik setzt ebenfalls Salizylsäure und AHA (Alpha-Hydroxy-Säuren) in der für Kosmetika zugelassenen niedrigen Konzentration ein, die jedoch zu gering sind, um tief im Ausführungsgang des Talgdrüsenhaarfollikel wirksam zu werden.

Zusätzlich werden Abrasiva zur Entfernung des obersten Hornpfropfen der Komeda angeboten.

### Sebosuppression

Die Sebosuppression bleibt aus rechtlichen Gründen vorwiegend der dermatologischen Therapie vorbehalten, wobei mit Antiandrogenen, Benzoylperoxid und Isotretinoin die Talgdrüsenproduktion effektiv gehemmt werden kann. Die Ergebnisse für kosmetisch zugängliche pflanzliche Phytohormone, zum Beispiel Kürbiskernextrakt oder Brenztraubensäure sind derzeit noch nicht gesichert, so dass nach dem Stand der Technik alle Versuche, die Komedonenbildung als ursächliches Problem zu lösen, zu wenig befriedigenden Ergebnissen geführt haben.

Unter neueren, aber ebenfalls der Dermatologie vorbehaltenen Stoffen, wird ein Retinoid der "dritten Generation" - "Adaptaben" genannt - verwendet, welches gezielt die entzündungsauslösenden Faktoren beeinflussen soll.

Neben den oben genannten Maßnahmen ist daher die topische Behandlung mit bakteriostatischen Mitteln unerlässlich, um die Kolonisation der Proplonibakterlen acnes auf der Haut zu verhindern oder zu vermindern.

Hierzu werden in der Vergangenheit in der Dermatologie topische und systemische Antibiotika, in erster Linie Erythromycin und Clindamycin eingesetzt, um synergetische Effekte zu erzielen, wobei in letzter Zeit immer deutlicher auf die Gefahren von Risiken der Resistenzblidung hingewiesen wurde. Diese Resistenzbildung soll durch die zusätzliche Kombination mit Benzoylperoxid und/oder Zink wirkungsvoll verhindert werden.

Wichtige topische und systemische Akne-Therapeutika und deren relevante Angriffspunkte in der Phatogenese sind somit:

| | topisch | systemisch |
|---|---|---|
| keratolytisch | Tretinoin | Isotretinoin |
| | Isotretinoin | |
| | Adapalen | |
| | Azeallnsäure | |
| | Benzoylperoxid | |
| | Salizylsäure | |
| antiseborrhoisch | alkoholische Lösung Syndets | Antiandrogene Isotretinoin |
| antimikrobiell/antiseptisch | Tetrazyline | Tetrazyline |
| | Erythromycin | - Tetrazylinhydrochlorid |
| | Clindamycin | - Doxyzylin |
| | Azealinsäure | - Minozylin |
| | Benzoylperoxid | |
| | Zink | Erythromycin |
| | Salizylsäure | Trimethoprim Sulfa- |
| | topisch | systemisch |
| antimikrobiell/antiseptisch | | Josamycin |
| | | Isotretinoin |
| | | Dapson |
| antientzündlich | UVB | Isotretinoin |
| | Azealinsäure | Dapson (Glucocorticosteroide) |

Eine gute Verträglichkeit spielt aber in der Behandlung mit kosmetischen Produkten eine Schlüsselrolle, abgesehen davon, dass der Kosmetik auch topische Antibiotika nicht zugänglich sind. In der Kosmetik werden daher eine Vielzahl antimikrobieller wirksamer Substanzen, sowohl synthetischer als auch natürlicher Herkunft eingesetzt, zum Beispiel nach der Kosmetikverordnung zugelassene Konservierungsstoffe. Bevorzugt sind es Triclosan oder ätherische Öle wie zum Beispiel Teebaumöl.

Diese werden wiederum häufig In Kombination mit Zink eingesetzt, das als klassische antimikrobiell wirksame Substanz bekannt ist. Nachteil der kosmetischen Zubereitungen ist es, dass die Einsatzkonzentration der gesetzlich zugelassenen antimikrobiell wirksamen Substanzen beschränkt ist, und damit vorwiegend zur Konservierung der Produkte dienen soll, andererseits die Gefahr von Allergien und zusätzlichen Entzündungen bei einer defekten Hornschicht der Haut möglich ist. Ebenso ist die Wirksamkeit auf das Propionibakterium auf wenige der zugelassenen Substanz beschränkt und mit Ausnahme von Triclosan wenig effektiv.

Es ist daher ein hohes Anliegen der Kosmetik eine wirksame und trotzdem hoch verträgliche Komponente oder Komposition zu finden, die sowohl den gesetzlichen Erfordernissen entspricht, als auch den Forderungen der Verbraucher nach Compliance und Behandlungserfolg.

Zur Lösung der zuvor geschilderten Aufgabe schlägt die vorliegende Erfindung eine Zubereitung nach Anspruch 1 vor; weitere vorteilhafte Ausgestaltungen sind Gegenstand der diesbezüglichen abhängigen Ansprüche.

Gegenstand der vorliegenden Erfindung ist somit eine bakteriostatische Zubereitung zur Vorbeugung und Behandlung der Akne und unreiner Haut, welche neben üblichen Hilfs- und Trägerstoffen
- ein Gemisch von Zinkglukonat und Kupferglukonat, wobei Zink und Kupfer, bezogen auf das Kation, im Molverhältnis 2 : 1 bis 20 : 1 vorliegen, sowie
- keratolytisch wirksame Substanzen und/oder Phytohormone
enthält.

Diese Kombination übt eine ausgeprägte antimikrobielle Wirkung auf die Propionibakterien aus, während den Einzelsubstanzen diese Wirkung nicht zukommt. Bevorzugt ist das Molverhältnis von etwa 2: 1 bis 10:1 Zink/Kupfer, besonders bevorzugt 4:1 bis 7: 1. So konnte mit dem Hemmhoftest gezeigt werden, dass eine ausgeprägte und spezifische Wirkung auf das Wachstum der Propionibakterie erzielt werden kann, die der des Triclosans gleichkommt, während dagegen andere Bakterienstämme nicht, oder kaum beeinflusst werden.

Während Triclosan im Körper die Leberfunktion beeinträchtigt, das Erbgut von Hefen schädigen kann und nach neuen Untersuchungen mögliche cancerogene Eigenschaften zugeschrieben werden, wurde damit eine natürliche Form der Bakteriostase der Propionibakterien gefunden, insbesondere da Zink und Kupfer als wichtige Mineralien des Organismus, insbesondere der Hautzellen gelten. Ebenso besteht bei Triclosan herstellungsbedingt die Gefahr durch Verunreinigungen wie Dioxine, die schon in geringer Menge auf der Haut Chlorakne auslösen können.

Aus der WO 01/00021 A1 sind fungizide und biocide Mittel bekannt enthaltend Pyrithione und Salze des Silbers, Zinkes und Kupfers, die auch in Shampoo eingesetzt werden können. Die Salze von Kupfer und Zink alleine werden jedoch als zu wenig wirksam angesehen.

Aus der WO 02/07700 A2 ist bekannt, oxidierte Kohlenhydrate einzusetzen, die Metallsalze enthalten. Diese Mittel sollen verwendet werden, den Haarwuchs zu regulieren

Die KR 2003-047457 A bzw. ihr entsprechendes Derwent-Abstract (DATABA-SE WPI, Section Ch, Week 200377, Derwent Publications Ltd., London, GB; Class B04, AN 2003-826265, XP002298897) betrifft kosmetische Zubereitungen auf Basis eines Extraktes des Sommer-Portulak *(Portulaca oleracea)* in Verbindung mit einem ternären System, bestehend aus Kupfer-, Zink- und Magnesiumglukonat, zur Vorbeugung und Behandlung von Akne.

Die WO 2004/022024 betrifft Mischungen von Sojaprodukten und organischen Salzen von Zink, Magnesium und Kupfer sowie die Verwendung dieser Mischungen in topisch angewendeten Zubereitungen, insbesondere in Anti-Aging-Produkten.

Die EP 1 444 973 A1 betrifft Mischungen von Wasser-Öl-Emulsionen mit für kosmetische und pharmazeutische Zwecke geeigneten Pulvern und die Verwendung dieser Mischungen als die Textur verändernde und prägende Mittel in kosmetischen und pharmazeutischen Erzeugnissen, wie z. B. Cremes, Lotionen und Pudern, aber auch zur Imprägnierung und Beschichtung von Papier und Textilien.

Schließlich betrifft die WO 03/013561 auf pflanzlichen Ausgangsstoffen basierende Zubereitungen zu kosmetischen und pharmazeutischen Zwecken, die oral bzw. systemisch angewendet werden und der Vorbeugung und Behandlung von Erkrankungen und Störungen der Kopfhaut dienen sollen. Die aktiven Komponenten in diesen Zubereitungen sind Extrakte der Sägepalme (*Serenoa repens*) und der Weinrebe *(Vitis vinifera).*

Die erfindungsgemäßen Zubereitungen auf Basis von Zinkglukonat und Kupferglukonat zeigen bezüglich der Toxizität sowie Haut- und Augenirritation, Sensibilisierung und Mutagenitätsdaten keine Auffälligkeiten. Zink und Kupfer liegen erfindungsgemäß in löslicher Form vor, nämlich als Glukonate. Bevorzugt wird somit eine Kombination aus 3-6 % Zinkgluconat und 0,1-1 % Kupfergluconat In leicht saurem Milieu (pH = 4-5). Dabei können die Salze auch in einer wässrigen alkoholischen oder wässrigen glykolischen Zubereitung vorgelöst werden. Eine solche Zubereitung A) weist beispielsweise folgende Zusammensetzung auf:
5 % Zinkgluconat
0,8 % Kupfergluconat
50 % Glycerin
100,0 cl Wasser
pH ca. 4,5

Diese Lösung kann mit üblichen Konservierungsmitteln stabilisiert werden, zum Beispiel mit Phenoxyethanol. Die Zubereitungen enthalten weiterhin typische Hilfs- und Trägerstoffe. Im folgenden sind einige typische Rezepturbeispiele zusammengestellt:

### Antibakterielles Gesichtswasser:

| Wasser | QSP | 100 % |
|---|---|---|
| Oleth 20 (Lösungsvermittler) | | 1-4 % |
| Glyzerin | | 2-7 % |
| Parfüm | | 0,2-1 % |
| Parabene | | 0,2-0,5 % |
| Minerallösung (A) 0,5-3 % | | |
| Salizylsäure | | 0,05-0,5 % |

### Antibakterielle Creme

| Wasser | QSP | 100 % |
|---|---|---|
| Glycerin | | 2-7 % |
| Methylmethacrylat crosspolymer | | 0,5-1,5 % |
| Minerallösung (A) | | 0,5-3 % |
| Cyclomethicone | | 1,5-2,5 % |
| Sodiumacrylat | | |
| Acryloyldimethyltaurat | | |
| Copolymer + Isohexadecan + Polhysorbat 80 | | 2,5-5 % |
| Parabene | | 0,2-0,5 % |
| Ginseng Wurzelextrakt | | 0,05-1 % |

### Antibakterielle Gesichtslotion

| Wasser | QSP | 100% |
|---|---|---|
| Glycerin | | 2-7% |
| Methylmethacrylat crosspolymer | | 0,5-1,5 |
| Minerallösung (A) | | 0,5-3 % |
| C14-22 Alkohol + C 12-20 Alkylglucoside | | 2-5 % |
| Isononylisonanoate | | 2-5 % |
| Sodiumacrylate / acrylolyldimethyl | | |
| taurat copolymer | | 0,5-1,5 % |
| + Isohexadecan + Polysorbat 80 | | |
| Kürbiskern-Extrakt | | 0,05-3 % |
| Cyclomethicone | | 10-15 % |
| Parfüm | | 0,1-1 % |
| Parabene | | 0,2-0,5 % |

### Antibakterielles Reinigungs-Fluid

| Wasser | QSP | 100% |
|---|---|---|
| Glycerin | | 2-8% |
| Parabene | | 0,2-0,5 % |
| Parfüm | | 0,1-1 % |
| Polysorbate 81 | | 1-3 % |
| Sodiumlauroyl OAT aminoacids | | 1-10 % |
| Sodiumlaurylethersulfate 28 % | | 20-50 % |
| Minerallösung (A) | | 0,5-3 % |
| Sodium Chlorid q.s | | |
| Lacticacid q.s | | |
| Cocoamideproylal - betainamide | | 2-5 % |
| Imidozolidinyl-urea | | 0,3 % |

## Patentansprüche

1. Bakteriostatische Zubereitung zur Vorbeugung und Behandlung der Akne und unreiner Haut, enthaltend neben üblichen Hilfs- und Trägerstoffen
• ein Gemisch von Zinkglukonat und Kupferglukonat, wobei Zink und Kupfer, bezogen auf das Kation, im Molverhältnis 2 : 1 bis 20 : 1 vorliegen, sowie
• keratolytisch wirksame Substanzen und/oder Phytohormone.

2. Zubereitung gemäß Anspruch 1, **dadurch gekennzeichnet, daß** pastöse oder flüssige Zubereitungen einen ein- oder mehrwertigen Alkohol enthalten und der pH-Wert im sauren Milieu liegt.

3. Zubereitung gemäß Anspruch 1 oder 2, **dadurch gekennzeichnet, daß** die Phytohormone androgene Hormone pflanzlichen Ursprungs sind.

4. Zubereitung gemäß einem der Ansprüche 1 bis 3, **dadurch gekennzeichnet, daß** die keratolytisch wirksamen Substanzen α- oder β-Hydroxysäuren oder deren Derivate sind.

## Claims

1. Bacteriostatic preparation for the prevention and treatment of acne and skin impurities, containing, in addition to conventional additives and carriers,
• a mixture of zinc gluconate and copper gluconate, wherein zinc and copper are present at a molar ratio of from 2:1 to 20:1, based on the cation; and
• keratolytically active substances and/or phytohormones.

2. Preparation according to claim 1, **characterised in that** viscous or liquid preparations contain a monohydric or polyhydric alcohol and the pH value is acidic.

3. Preparation according to either claim 1 or claim 2, **characterised in that** the phytohormones are androgenic hormones of plant origin.

4. Preparation according to any one of claims 1 to 3, **characterised in that** the keratolytically active substances are α- or β-hydroxy acids or derivatives thereof.

## Revendications

1. Préparation bactériostatique pour la prévention et le traitement de l'acné et des impuretés de la peau, contenant, outre les substances auxiliaires et porteuses usuelles:
• un mélange de gluconate de zinc et de gluconate de cuivre, le zinc et le cuivre étant présents dans une proportion molaire de 2:1 à 20:1 par rapport au cation ainsi que
• des substances et/ou phytohormones actives au niveau kératolytique.

2. Préparation selon la revendication 1, **caractérisée en ce que** les préparations pâteuses ou liquides contiennent un alcool uni- ou polyvalent et que la valeur pH se situe dans le milieu acide.

3. Préparation selon la revendication 1 ou 2, **caractérisée en ce que** les phytohormones sont des hormones androgènes d'origine végétale.

4. Préparation selon une des revendications 1 à 3, **caractérisée en ce que** les substances actives au niveau kératolytique sont des α- ou β-hydroxyacides ou leurs dérivés.
